(19) Europäisches Patentamt
European Patent Office
Office européen des brevets

(11) Veröffentlichungsnummer: **0 389 426 A1**

# (12) EUROPÄISCHE PATENTANMELDUNG

(21) Anmeldenummer: **90810194.2**

(22) Anmeldetag: **13.03.90**

(51) Int. Cl.5: **C07D 417/04, C07D 413/04, C07D 417/14, C07D 413/14, A01N 43/82**

(30) Priorität: **21.03.89 CH 1038/89**
**23.01.90 CH 195/90**

(43) Veröffentlichungstag der Anmeldung:
**26.09.90 Patentblatt 90/39**

(84) Benannte Vertragsstaaten:
**AT BE CH DE DK ES FR GB GR IT LI LU NL**

(71) Anmelder: **CIBA-GEIGY AG**
**Klybeckstrasse 141**
**CH-4002 Basel(CH)**

(72) Erfinder: **Eckhardt, Wolfgang, Dr.**
**Breslauerstrasse 14**
**D-7850 Lörrach(DE)**
Erfinder: **Beriger, Ernst, Dr.**
**Grabenmattweg 29**
**D-4123 Allschwil(DE)**

## (54) Nematizide und fungizide Mittel.

(57) Es werden 2-Mercapto-5-thiazol-(2-, 4- oder 5-yl)-1,3,4-oxa- und -thiadiazole und 2-Mercapto-5-oxazol-(2-, 4- oder 5-yl)-1,3,4-oxa- und -thiadiazole der Formel I

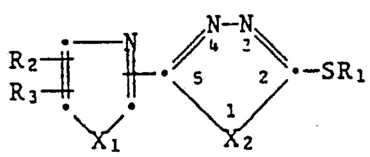

(I)

in welcher $X_1$ und $X_2$ unabhängig voneinander Sauerstoff oder Schwefel $R_1$ unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl, $R_2$, $R_3$ unabhängig voneinander Wasserstoff, $C_1$-$C_5$-Alkyl, unsubstituiertes oder durch Halogen substituiertes $C_3$-$C_7$-Alkenyl, unsubstituiertes oder durch $C_1$-$C_3$-Alkyl, $C_1$-$C_3$-Alkoxy oder Halogen gleich oder verschieden substituiertes Phenyl, den Rest $-NR_4R_5$, wobei $R_4$ und $R_5$ unabhängig voneinander für Wasserstoff, $C_1$-$C_5$-Alkyl, unsubstituiertes oder durch Halogen substituiertes $C_3$-$C_7$-Alkenyl stehen, oder einer der beiden Substituenten $R_2$ oder $R_3$ auch einen unsubstituierten oder substituierten Heterocyclen Pyridyl, Thienyl, Furyl oder Thiazolyl bedeuten, Verfahren zur Herstellung der Verbindungen der Formel I, sowie neue Zwischenprodukte des Herstellungsverfahrens beschrieben.

Die Verbindungen der Formel I besitzen nematizide und fungizide Eigenschaften. Es werden nematizide und fungizide Mittel, die als Wirkstoff mindestens einen Wirkstoff der Formel I enthalten, ferner Verfahren zur Verwendung der Wirkstoffe und der Mittel bei der Bekämpfung von Nematoden und Pilzen beschrieben.

EP 0 389 426 A1

## Nematizide und fungizide Mittel

Die vorliegende Erfindung betrifft neue substituierte Oxa- und Thiadiazole, deren Herstellung sowie nematizide Mittel, die als Wirkstoff mindestens eine dieser Verbindungen enthalten. Die Erfindung betrifft ferner neue Zwischenprodukte des Verfahrens zur Herstellung der Wirkstoffe, ihre Verwendung und Mittel zur Bekämpfung von Nematoden und Pilzen, insbesondere von pflanzenschädigenden Nematoden und Pilzen.

Die Erfindung betrifft 2-Mercapto-5-thiazol-(2-, 4- oder 5-yl)-1,3,4-oxa-und -thiadiazole und 2-Mercapto-5-oxazol-(2-, 4- oder 5-yl)-1,3,4-oxa-und -thiadiazole der Formel I

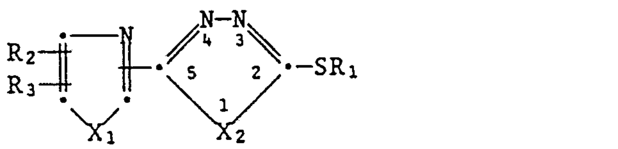

(I)

in welcher bedeuten:

$X_1$ und $X_2$ unabhängig voneinander Sauerstoff oder Schwefel $R_1$ unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl, $R_2$, $R_3$ unabhängig voneinander Wasserstoff, $C_1$-$C_5$-Alkyl, unsubstituiertes oder durch Halogen substituiertes $C_3$-$C_7$-Alkenyl, unsubstituiertes oder durch $C_1$-$C_3$-Alkyl, $C_1$-$C_3$-Alkoxy oder Halogen gleich oder verschieden substituiertes Phenyl, den Rest $-NR_4R_5$, wobei $R_4$ und $R_5$ unabhängig voneinander für Wasserstoff, $C_1$-$C_5$-Alkyl, unsubstituiertes oder durch Halogen substituiertes $C_3$-$C_7$-Alkenyl stehen, oder einer der beiden Substituenten $R_2$ oder $R_3$ auch einen der heterocyclischen Reste Pyridyl, Thienyl, Furyl oder Thiazolyl bedeutet, der unsubstituiert oder durch $C_1$-$C_3$-Alkyl, $C_1$-$C_3$-Alkoxy oder Halogen ein-oder mehrfach gleich oder verschieden substituiert sein kann; sowie Salze dieser Verbindungen.

Unter Alkyl sind als selbständiger Rest sowie als Teil einer anderen Gruppe, wie Alkoxy, gerad- und verzweigtkettige Alkylgruppen zu verstehen. Dazu zählen die Methyl-, die Ethyl- sowie die normalen und isomeren Propyl-, Butyl- und Pentylgruppen. Halogensubstituiertes Alkyl steht für einen einfach bis perhalogenierten Alkylrest, wie z.B. $CHCl_2$, $CH_2F$, $CCl_3$, $CH_2Cl$; $CHFCH_3$, $CH_2CH_2Br$, $CF_2CF_3$, $C_2Cl_5$, $CH_2Br$, $CHBrCl$ usw., vorzugsweise für $CHF_2$. Alkenyl bedeutet z.B. Propenyl-(1), Allyl, Butenyl-(1), Butenyl-(2) oder Butenyl-(3) sowie Ketten mit mehreren Doppelbindungen. Alkinyl bedeutet z.B. Propinyl-(2), Butinyl-(1), Butinyl-(2), Pentinyl-(4) usw.. Halogen steht für Fluor, Chlor, Brom oder Jod, vorzugsweise für Fluor, Chlor oder Brom.

Der als Substituent genannte Pyridylrest kann durch den 2-, 3- oder 4-stelligen Kohlenstoff, die Furyl- oder Thienylreste können durch den 2-oder 3-stelligen Kohlenstoff und der Thiazolylrest durch den 2-, 4- oder 5-stelligen Kohlenstoff an den Oxa- oder Thiazolring gebunden sein.

Beispiele salzbildender Säuren sind unter den anorganischen Säuren Halogenwasserstoffsäuren wie Fluorwasserstoffsäure, Chlorwasserstoffsäure, Bromwasserstoffsäure oder Jodwasserstoffsäure sowie ferner Schwefelsäure, Phosphorsäure, phosphorige Säure, Salpetersäure und unter den organischen Säuren Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Es sind bereits Oxadiazol- und Thiadiazol-Derivate bekannt, die als nematizid wirksam beschrieben sind. So sind in der US-Patentschrift 3,770,754 solche Verbindungen mit einer 1,2,4-Stellung der Heteroatome offenbart, während in der US-Patentschrift 4,454,147 1,3,4-Thiadiazol-Derivate beschrieben sind, bei denen im Vergleich mit den erfindungsgemässen Verbindungen der Heterocyclus anstelle der Mercapto-Gruppen durch ein Chloratom substituiert ist. Diese bekannten Verbindungen haben bisher als Nematizide die in der Praxis an sie gestellten Ansprüche nicht in vollem Umfang befriedigen können. Ferner sind Oxadiazol-Derivate mit fungizider Wirksamkeit in der DE-OS 2 361 613 beschrieben. Es werden darin jedoch keine dieser Verbindungen expressis verbis genannt, die unter den Umfang der erfindungsgemässen Formel I fallen.

Durch die Bereitstellung der erfindungsgemässen Verbindungen der Formel I ist es nun gelungen, einen wertvollen Beitrag zur Bekämpfung der beträchtliche landwirtschaftliche Schäden an Pflanzgut

verursachenden Pflanzennematoden und Pilzen zu leisten. Auf diese Weise können Ernteeinbussen bei Kulturpflanzen wie z.B. Kartoffeln, Getreide, Rüben, Raps, Kohl, Tabak, Sojabohnen, Baumwolle, Mais, Reis und Gemüse sowie Schäden in Baumschulen und im Zierpflanzenbau nachhaltig eingedämmt werden. Die erfindungsgemässen Verbindungen zeichnen sich dabei insbesondere in der wirkungsvollen Bekämpfung von wurzelparasitären Bodennematoden wie z.B. solchen der Gattungen Heterodera und Globodera (zystenbildende Nematoden), Meloidogyne (Wurzelgallennematoden) sowie der Gattungen Radopholus, Pratylenchus, Tylenchulus, Longidorus, Trichodorus und Xiphinema aus. Ferner lassen sich mit den erfindungsgemässen Wirkstoffen die Nematodengattungen Ditylenchus (Stengelparasiten) Aphelenchoides (Blattnematoden) und Anguina (Blütennematoden) wirkungsvoll bekämpfen.

Mit den Wirkstoffen der Formel I lassen sich vorzugsweise besonders schädliche Nematodenarten der Gattung Meloidogyne, wie z.B. Meloidogyne incognita, sowie der Gattung Heterodera, wie z.B. Heterodera glycines (Sojabohnen-Zystennematode) und ferner der Gattung Globodera, wie z.B. Globodera rostochiensis (Kartoffelzystennematode) sowie Vertreter von wandernden Endoparasiten, wie z.B. Pratylenchus penetrans oder Radopholus similis und Vertreter von Ektoparasiten, wie z.B. Trichodorus spp. und Xiphinema spp. erfolgreich bekämpfen.

Ebenso lassen sich vorzugsweise mit den Wirkstoffen der Formel I besonders schädliche Bodenpilze der Gattung Pythium, wie z.B. Pythium ultimum, sowie der Gattung Rhizoctonia, wie z.B. Rhizoctonia solani erfolgreich bekämpfen.

Zur Bekämpfung der Pflanzennematoden und Bodenpilze und zur Gesunderhaltung des Pflanzgutes können die neuen Wirkstoffe kurativ, präventiv oder systemisch eingesetzt werden. Dabei entfalten sie eine breit gefächerte Aktivität gegen die verschiedenen Nematoden- und Bodenpilzspecies und werden somit den Erfordernissen der Praxis gerecht. Die nematizide und fungizide Wirkungsweise der erfindungsgemässen Verbindungen wird durch eine geringe Phytotoxizität in vorteilhafter Weise begleitet, womit der allgemein wünschenswerten Verminderung der Umweltbelastung in besonderem Masse Rechnung getragen wird.

Eine bevorzugte Untergruppe sind Verbindungen der Formel 7, worin $X_1$ und $X_2$ unabhängig voneinander O oder S, und $R_1$ durch Halogen substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl bedeuten; $R_2$ Wasserstoff $C_1$-$C_3$ Alkyl ist; $R_3$ Wasserstoff, $C_1$-$C_3$-Alkyl, unsubstituiertes oder durch $C_1$-$C_3$-Alkyl, $C_1$-$C_3$-Alkoxy oder Halogen gleich oder verschieden substituiertes Phenyl oder den Rest $-NR_4R_5$ bedeutet, oder einer der beiden Substituenten $R_2$ oder $R_3$ auch einen der Heterocyclen Pyridyl, Thienyl, Furyl oder Thiazolyl bedeutet, der unsubstituiert oder wahlweise durch $C_1$-$C_3$-Alkyl, $C_1$-$C_3$-Alkoxy oder Halogen gleich oder verschieden ein- oder zweifach substituiert sein kann.

Von dieser Gruppe sind jene Verbindungen bedeutend, bei welchen $X_1$ und $X_2$ unabhängig voneinander Sauerstoff oder Schwefel und $R_1$ $C_1$-$C_5$-Haloalkyl; unsubstituiertes oder durch Halogen substituiertes $C_3$-$C_7$-Alkenyl; unsubstituiertes oder durch Halogen substituiertes $C_3$-$C_7$-Alkinyl bedeuten; $R_2$ Wasserstoff oder $C_1$-$C_3$-Alkyl ist; $R_3$ Wasserstoff, $C_1$-$C_5$-Alkyl, unsubstituiertes oder durch $C_1$-$C_3$-Alkyl, $C_1$-$C_3$-Alkoxy oder Halogen gleich oder verschieden substituiertes Phenyl bedeutet, oder einer der beiden Substituenten $R_2$ oder $R_3$ auch einen der Heterocyclen Pyridyl, Thienyl, Furyl oder Thiazolyl bedeutet, der unsubstituiert oder wahlweise durch $C_1$-$C_3$-Alkyl, $C_1$-$C_3$-Alkoxy oder Halogen gleich oder verschieden ein- oder zweifach substituiert sein kann.

Weiterhin sind Verbindungen der Formel I bevorzugt, in welcher $X_1$ und $X_2$ unabhängig voneinander Sauerstoff oder Schwefel, $R_1$ unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl; $R_2$ und $R_3$ unabhängig voneinander Wasserstoff, $C_1$-$C_3$-Alkyl, unsubstituiertes oder durch $C_1$-$C_3$-Alkyl, $C_1$-$C_3$-Alkoxy oder Halogen substituiertes Phenyl, Pyridyl, Thienyl, Furyl oder Thiazolyl bedeuten.

Im Rahmen der vorliegenden Erfindung sind weiterhin jene 2-Mercapto-5-thiazol-(2-, 4- oder 5-yl)-1,3,4-oxadiazole der Formel Ia

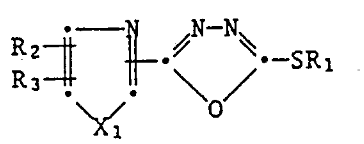

(Ia)

bevorzugt, bei welchen $R_1$ Difluormethyl, 3,4,4-Trifluor-3-buten Cyanomethyl, Difluormethyl-difluormethyl oder Propargyl, $R_2$ und $R_3$ unabhängig voneinander Wasserstoff oder Methyl bedeuten.

Von diesen Verbindungen sind folgende Einzelverbindungen insbesondere bevorzugt:
2-Difluormethylthio-5-(thiazol-2-yl)-1,3,4-oxadiazol,
2-(3,4,4-Trifluor-3-butenylthio)-5-(thiazol-2-yl)-1,3,4-oxadiazol,
2-Difluormethylthio-5-(2-methyl-thiazol-4-yl)-1,3,4-oxadiazol,
2-(3,4,4-Trifluor-3-butenylthio)-5-(2-methyl-thiazol-4-yl)-1,3,4-oxadiazol,
2-(3,4,4-Trifluor-3-butenylthio)-5-[2-(pyrid-3-yl)-thiazol-4-yl]-1,3,4-oxadiazol,
2-Difluormethylthio-5-(thiazol-5-yl)-1,3,4-oxazol,
2-(3,4,4-Trifluor-3-butenylthio)-5-(thiazol-5-yl)-1,3,4-oxadiazol.

Bevorzugt sind auch jene 2-Mercapto-5-oxazol-(2-, 4- oder 5-yl)-1,3,4-thiadiazole der Formel Ib

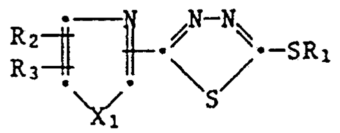

(Ib)

bei welchen $R_1$ Difluormethyl, 3,4,4-Trifluor-3-buten, Tetrafluoräthyl, Cyanomethyl, Allyl, Propargyl oder 2-Bromalkyl, $R_2$ und $R_3$ unabhängig voneinander Wasserstoff oder Methyl bedeuten.

Von dieser Gruppe sind folgende Einzelverbindungen insbesondere bevorzugt:
2-Difluormethylthio-5-(thiazol-2-yl)-1,3,4-thiadiazol,
2-(3,4,4-Trifluor-3-butenylthio)-5-(thiazol-2-yl)-1,3,4-thiadiazol,
2-Difluormethylthio-5-(2-methyl-thiazol-4-yl)-1,3,4-thiadiazol,
2-(3,4,4-Trifluor-3-butenylthio)-5-(2-methylthiazol-4-yl)-1,3,4-thiadiazol,
2-Difluormethylthio-5-(thiazol-5-yl)-1,3,4-thiadiazol,
2-(3,4,4-Trifluor-3-butenylthio)-5-(thiazol-5-yl)-1,3,4-thiadiazol.

Verbindungen der Formel I werden erfindungsgemäss hergestellt, indem man

a) in einer Kondensationsreaktion eine Verbindung der Formel II

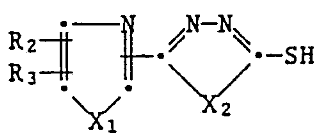

(II)

oder eine Verbindung der Formel III

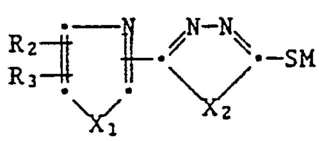

(III)

mit einer Verbindung der Formel IV

Hal-$R_1$ (IV)

in einem inerten Lösungsmittel oder Lösungsmittel-Gemisch bei Raumtemperatur oder bei erhöhter Temperatur gegebenenfalls in Gegenwart eines Katalysators und gegebenenfalls bei erhöhtem Druck umsetzt, wobei die Umsetzung einer Verbindung der Formel II in Gegenwart einer Base verläuft, oder

b) in einer Anlagerungsreaktion eine Verbindung der Formel II

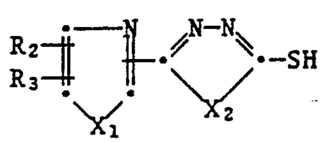

(II)

mit einer Verbindung der Formel V

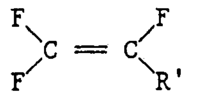

(V)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ic

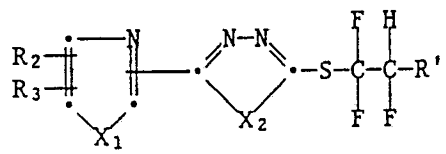

(Ic)

oder zu einer Verbindung der Formel Id

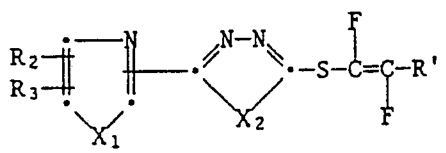

(Id)

führt, wobei in den vorstehend genannten Formeln II, III, Ic, Id, IV und V M für ein Alkalimetall oder für Ammonium steht, Hal Halogen, vorzugsweise Chlor, Brom oder Jod bedeutet, und $R'$ Fluor oder Trifluormethyl darstellt, während $R_1$, $R_2$, $R_3$, $X_1$ und $X_2$ die unter Formel I angegebenen Bedeutungen besitzen.

Als Alkalimetalle für den Rest M kommen beispielsweise Kalium und Natrium in Frage; der Ammoniumrest kann organische Reste, wie beispielsweise den Trialkylammoniumrest auch bedeuten.

Für die Herstellung der erfindungsgemässen Wirksubstanzen geeignete Lösungs- oder Verdünnungsmittel sind z.B. Ether und etherartige Verbindungen wie Dialkylether (Diethylether, Diisopropylether, tert.-Butylmethylether usw.), Anisol, Dioxan, Tetrahydrofuran; aliphatische und aromatische Kohlenwasserstoffe wie Benzol, Toluol, Petrolether; halogenierte Kohlenwasserstoffe wie Chlorbenzol, Methylenchlorid, Chloroform, Ethylenchlorid, Tetrachlorkohlenstoff, Tetrachlorethylen; Nitrile wie Acetonitril, Propionitril; N,N-dialkylierte Amide wie Dimethylformamid; Dimethylsulfoxid; Ketone wie Aceton, Diethylketon, Methylethylketon sowie Wasser und Alkohole wie z.B. Methanol, Ethanol, Isopropanol oder Butanol; und ganz allgemein Gemische solcher Lösungsmittel untereinander.

Als Basen kommen organische und anorganische Basen in Betracht; z.B. vorzugsweise tertiäre Amine wie Trialkylamine (Trimethylamin, Triethylamin, Tripropylamin usw.), sowie Oxide, Hydroxide, Carbonate und Hydrogencarbonate von Alkali- und Erdalkalimetallen (z.B. CaO, BaO, NaOH, KOH, $Ca(OH)_2$, $KHCO_3$, $NaHCO_3$, $Ca(HCO_3)_2$, $K_2CO_3$, $Na_2CO_3$ usw.), ferner Acetate wie z.B. $CH_3COONa$ oder $CH_3COOK$. Darüber hinaus eignen sich als Basen auch Alkalialkoholate wie z.B. Natriumethylat, Natriumpropylat, Kalium-tert.-butylat oder Natriummethylat.

Die Zugabe katalytischer Mengen eines Kronenethers, wie z.B. 18-Krone-6 oder 15-Krone-5, wirkt sich in den Herstellungsverfahren günstig auf den Reaktionsablauf aus. Ferner hat sich für den gleichen Zweck die katalytische Verwendung von Tetraalkylaminsalzen, z.B. Tetraalkylammoniumchloride oder -bromide, vorzugsweise Tetra-n-butylammoniumbromid oder Tetramethylammoniumchlorid, als vorteilhaft erwiesen. Darüber hinaus sind Alkalijodide, vorzugsweise Kaliumjodid, als Katalysatoren vorteilhaft einsetzbar.

In den Herstellungsverfahren betragen die Reaktionstemperaturen 10 bis 90° C, vorzugsweise 30° bis 80° C. Für die Druckverhältnisse während des Reaktionsablaufs sind 1 bis 20 bar, vorzugsweise 6 bis 14 bar, massgebend.

Die Ausgangsverbindungen der Formel II sind teils bekannt und können nach bekannten Verfahren hergestellt werden [G.B. 845 715, Egypt J. Chem. 29, 259 - 263 (1986) C.A. 109 (19) 170319u, C.A. 186358s]; sie weisen keine nematiziden Eigenschaften auf.

Die Erfindung betrifft auch Mittel, zur Bekämpfung von pflanzenschädigenden Nematoden und Pilzen sowie zur präventiven Verhütung des Nematoden- und Pilzbefalls von Pflanzgut, welche die Wirkstoffe der Formel I enthalten.

Darüber hinaus schliesst die vorliegende Erfindung zusätzlich die Herstellung nematizider und fungizider Mittel ein, die gekennzeichnet ist durch das innige Vermischen von Aktivsubstanzen der Formel I mit

einer oder mehreren hierin beschriebenen Trägerstoffen und Hilfsingredienzen. Eingeschlossen ist auch ein Verfahren zur Behandlung von Pflanzen, das durch die Applikation der Verbindungen der Formel I oder der neuen Mittel charakterisiert ist.

Ein bezvorzugtes Verfahren für den Einsatz eines Wirkstoffs der Formel I bzw. eines nematiziden und fungiziden Mittels, das mindestens einen dieser Wirkstoffe enthält, ist das Einbringen in den Erdboden. Dabei wird der Standort der Pflanzen mit einer flüssigen oder festen Zubereitung behandelt.

Die Verbindungen der Formel I können aber auch auf Samenkörner aufgebracht werden (Beizung/Coating), indem man die Körner entweder in einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel, Knospen oder Blätter.

Wirkstoffe der Formel I werden üblicherweise in Form von formulierten Zusammensetzungen verwendet und können gleichzeitig oder nacheinander mit weiteren Wirkstoffen auf die zu behandelnde Fläche oder Pflanze gegeben werden. Diese weiteren Wirkstoffe können auch andere in der Landwirtschaft angewendete Mittel umfassen, die in ihrer Nutzanwendung der Produktionssteigerung durch Förderung des Nutzpflanzenwachstums dienen, wie Düngemittel, Herbizide, Insektizide, Fungizide, Molluskizide u.a., oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel I werden in unveränderter Form oder vorzugsweise zusammen mit den in der Formulierungstechnik üblichen Hilfsmitteln eingesetzt. Sie werden z.B. zu Emulsionskonzentraten, direkt versprüh baren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Verstäuben, Verstreuen oder Giessen werden gleich wie die Art der Mittel den angestrebten Zielen und den gegebenen Verhältnissen entsprechend gewählt. Günstige Aufwandmengen liegen im allgemeinen bei 500 g bis 6 kg Aktivsubstanz (AS) je ha; bevorzugt 1 bis 4 kg AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenfalls mit oberflächenaktiven Substanzen (Tensiden).

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Substanzen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na-oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch

den Alkylteil von Acylresten einschliesst, z.B. Das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von FettalkoholEthylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die neuen Formulierungstechnik gebräuchlichen Tenside sind u.a. in folgenden Publikationen beschrieben:
"Mc Cutcheon's Detergents and Emulsifiers Annual"
MC Publishing Corp., Ridgewood, New Jersey, 1979;
Dr. Helmut Stache "Tensid Taschenbuch", Carl Hanser Verlag München/Wien.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken.

## 1. Herstellungsbeispiele

### 2-Difluormethylthio-5-(2-methyl-thiazol-4-yl)-1,3,4-oxadiazol (Verb. 2.6)

2,4 g (0,0369 Mol) 85 %iges Kaliumhydroxid werden in 12 ml Wasser gelöst und mit 4,9 g (0,0246 Mol) 2-Mercapto-5-(2-methylthiazol-4-yl)-1,3,4-oxadiazol, 2,5 g Tetramethylammoniumchlorid und 120 ml 1,4-Dioxan versetzt. In das kräftig gerührte Reaktionsgemisch werden während 8 Stunden 3,2 g (0,037 Mol) Freon 22 bei Zimmertemperatur eingeleitet. Nun werden weitere 1,2 g (0,018 Mol) 85%iges Kaliumhydroxid und 1,2 g Tetramethylammoniumchlorid zugegeben und während weiteren 8 Stunden 1,6 g (0,018 Mol) Freon 22 bei 25°C eingeleitet. Das Lösungsmittel wird unter vermindertem Druck abdestilliert und der Rückstand in 100 ml Essigsäureethylester gelöst und nacheinander je 3 mal mit je 50 ml 1N Natronlauge und Wasser extrahiert. Die Essigsäureethylesterlösung wird mit Natriumsulfat getrocknet, filtriert und unter

vermindertem Druck eingeengt. Man erhält so 0,6 g Produkt vom Smp. 82 - 85 °C.

<u>2-(3,4,4-Trifluor-3-butenylthio)-5-(2-methylthiazol-4-yl)-1,3,4-thiadiazol (Verb. 2.10)</u>

3 g (0,014 Mol) 2-Mercapto-5-(2-methylthiazol-4-yl)-1,3,4-thiadiazol werden in einem Gemisch von 100 ml 1,4-Dioxan und 10 ml Dimethylformamid gelöst und mit 1,7 g (0,0154 Mol) Kalium-tert-butylat versetzt. Nun werden 4 g (0,021 Mol) 4-Brom-1,1,2-trifluorbuten bei 25 °C zugetropft und das Reaktionsgemisch 16 Std. bei 25 °C gerührt. Nun wird das Lösungsmittel unter vermindertem Druck abdestilliert und der Rückstand in ca. 100 ml Essigsäureethylester gelöst und nacheinander je 3 mal mit je ca. 50 ml 1N Natronlauge und Wasser extrahiert. Die Essigsäureethylesterlösung wird mit Natriumsulfat getrocknet, filtriert und unter vermindertem Druck eingeengt. Man erhält so 2,9 g Produkt vom Smp. 65 - 68 °C.

Tabelle 1, Oxa- und Thiazol-2-yl-Derivate

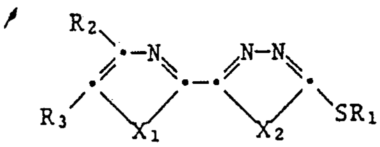

| Verb. Nr. | $R_1$ | $R_2$ | $R_3$ | $X_1$ | $X_2$ | Physikalische Daten |
|---|---|---|---|---|---|---|
| 1.1 | H | H | H | S | O | Smp. 192-195°C |
| 1.2 | H | H | H | S | S | ab 125°C Zers. |
| 1.3 | $-CH_3$ | H | H | S | O | |
| 1.4 | $-CF_3$ | H | H | S | O | |
| 1.5 | $-CF_2CHF_2$ | H | H | S | O | Smp. 98-102°C |
| 1.6 | $-CHF_2$ | H | H | S | O | Smp. 91-93°C |
| 1.7 | $-CHF_2$ | H | H | S | S | |
| 1.8 | $-CHF_2$ | $CH_3$ | H | S | O | |
| 1.9 | $-CHF_2$ | $CH_3$ | H | S | S | |
| 1.10 | $-CHF_2$ | H | $CH_3$ | S | O | |
| 1.11 | $-CHF_2$ | H | $CH_3$ | S | S | |
| 1.12 | $-CHF_2$ | $CH_3$ | $CH_3$ | S | O | |
| 1.13 | $-CHF_2$ | $CH_3$ | $CH_3$ | S | S | |
| 1.14 | $-CHF_2$ | H | H | O | O | |
| 1.15 | $-CHF_2$ | H | H | O | S | |
| 1.16 | $-CHF_2$ | $CH_3$ | H | O | O | |
| 1.17 | $-CHF_2$ | $CH_3$ | H | O | S | |
| 1.18 | H | H | H | O | O | |
| 1.19 | H | H | H | O | S | |
| 1.20 | H | $CH_3$ | H | O | O | |
| 1.21 | H | $CH_3$ | H | O | S | |
| 1.22 | $-CH_2CH_2CF=CF_2$ | H | H | S | O | $n_D^{30}$: 1,5683 |
| 1.23 | $-CH_2CH_2CF=CF_2$ | H | H | S | S | $n_D^{30}$: 1,6088 |
| 1.24 | $-CH_2CH_2CF=CF_2$ | $CH_3$ | H | S | O | |
| 1.25 | $-CH_2CH_2CF=CF_2$ | $CH_3$ | H | S | S | |
| 1.26 | $-CH_2CH_2CF=CF_2$ | $CH_3$ | H | O | O | |
| 1.27 | $-CH_2CH_2CF=CF_2$ | $CH_3$ | H | O | S | |
| 1.28 | $-CH_2CH_2CF=CF_2$ | H | $CH_3$ | S | O | |
| 1.29 | $-CH_2CH_2CF=CF_2$ | $CH_3$ | $CH_3$ | S | O | |
| 1.30 | $-CH_2CH_2CF=CF_2$ | $CH_3$ | $CH_3$ | O | O | |
| 1.31 | $-CH_2CH=CH_2$ | H | H | S | O | |
| 1.32 | $-CH_2CH=CH_2$ | H | H | O | O | |
| 1.33 | $-CH_2CH=CH_2$ | H | H | S | S | |
| 1.34 | $-CH_2CH=CH_2$ | H | H | O | S | |
| 1.35 | $-CH_2CH{\equiv}CH$ | H | H | S | O | |
| 1.36 | $-CH_2CH{\equiv}CH$ | H | H | S | S | |
| 1.37 | $-CH_2CH{\equiv}CH$ | H | H | O | S | |
| 1.38 | $-CH_2CH{\equiv}CH$ | H | H | O | O | |
| 1.39 | $-CH_2CN$ | H | H | S | O | |
| 1.40 | $-CH_2CN$ | H | H | O | O | |

Tabelle 2, Oxa- und Thiazol-4-yl-Derivate

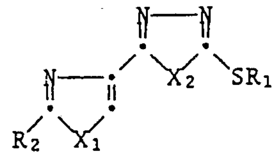

| Verb. Nr. | $R_1$ | $R_2$ | $X_1$ | $X_2$ | Physikalische Daten |
|---|---|---|---|---|---|
| 2.1 | H | $CH_3$ | S | O | Smp. 198-202°C |
| 2.2 | H | $CH_3$ | O | O | |
| 2.3 | H | $CH_3$ | O | S | |
| 2.4 | H | $CH_3$ | S | S | Smp. (Zers.) ab 143°C |
| 2.5 | $-CHF_2$ | $CH_3$ | O | O | |
| 2.6 | $-CHF_2$ | $CH_3$ | S | O | Smp. 82-85°C |
| 2.7 | $-CHF_2$ | $CH_3$ | O | S | |
| 2.8 | $-CHF_2$ | $CH_3$ | S | S | Smp. 98-100°C |
| 2.9 | $-CH_2CH_2CF=CF_2$ | $CH_3$ | O | O | |
| 2.10 | $-CH_2CH_2CF=CF_2$ | $CH_3$ | S | S | Smp. 65-68°C |
| 2.11 | $-CH_2CH_2CF=CF_2$ | $CH_3$ | O | S | |
| 2.12 | $-CH_2CH_2CF=CF_2$ | $CH_3$ | S | O | $n_D^{50}$ 1,5482 |
| 2.13 | $-CH_2CH_2CF=CF_2$ | H | S | O | |
| 2.14 | $-CH_2CH_2CF=CF_2$ | H | S | S | |
| 2.15 | $-CH_2CH_2CF=CF_2$ | $C_2H_5$ | S | O | |
| 2.16 | $-CH_2CH_2CF=CF_2$ | $C_2H_5$ | S | S | |
| 2.17 | $-CH_2CH_2CF=CF_2$ | $C_3H_7(n)$ | S | S | |
| 2.18 | $-CH_2CH_2CF=CF_2$ | $C_3H_7(n)$ | S | O | |
| 2.19 | $-CH_2CH_2CF=CF_2$ | $C_3H_7(i)$ | S | S | |
| 2.20 | $-CH_2CH_2CF=CF_2$ | $C_3H_7(i)$ | S | O | |
| 2.21 | $-CHF_2$ | H | S | O | |
| 2.22 | $-CHF_2$ | H | S | S | |
| 2.23 | $-CHF_2$ | $C_2H_5$ | S | O | |
| 2.24 | $-CHF_2$ | $C_2H_5$ | S | S | |
| 2.25 | $-CHF_2$ | $C_3H_7(n)$ | S | O | |
| 2.26 | $-CHF_2$ | $C_3H_7(n)$ | S | S | |
| 2.27 | $-CHF_2$ | $C_3H_7(i)$ | S | O | |
| 2.28 | $-CHF_2$ | $C_3H_7(i)$ | S | S | |
| 2.29 | H | $C_6H_5$ | S | O | |
| 2.30 | H | $C_6H_5$ | S | S | |
| 2.31 | $-CHF_2$ | $C_6H_5$ | S | O | |
| 2.32 | $-CHF_2$ | $C_6H_5$ | S | S | |
| 2.33 | $-CH_2CH_2CF=CF_2$ | $C_6H_5$ | S | O | |
| 2.34 | $-CH_2CH_2CF=CF_2$ | $C_6H_5$ | S | S | |
| 2.35 | H | 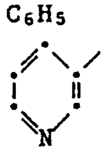 | S | O | Smp. > 270°C |
| 2.36 | H | 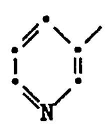 | S | S | Smp. > 260°C |

Tabelle 2 (Fortsetzung)

| Verb. Nr. | $R_1$ | $R_2$ | $X_1$ | $X_2$ | Physikalische Daten |
|---|---|---|---|---|---|
| 2.37 | $-CHF_2$ | 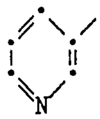 | S | O | |
| 2.38 | $-CHF_2$ | 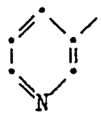 | S | S | Smp.122-124°C |
| 2.39 | $-CH_2CH_2CF=CF_2$ | 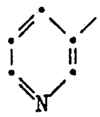 | S | O | Smp.106-108°C |
| 2.40 | $-CH_2CH_2CF=CF_2$ | 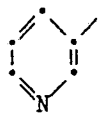 | S | S | Smp.125-130°C |
| 2.41 | H | 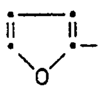 | S | O | |
| 2.42 | H | 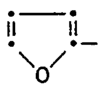 | S | S | |
| 2.43 | $-CHF_2$ | 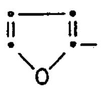 | S | O | |
| 2.44 | $-CHF_2$ | 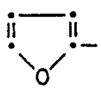 | S | S | |
| 2.45 | $-CH_2CH_2CF=CF_2$ | 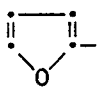 | S | O | |
| 2.46 | $-CH_2CH_2CF=CF_2$ | 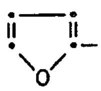 | S | S | |
| 2.47 | H | 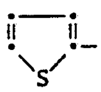 | S | O | |
| 2.48 | H | 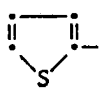 | S | S | |
| 2.49 | $-CHF_2$ | 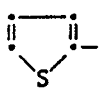 | S | O | |
| 2.50 | $-CHF_2$ | 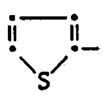 | S | S | |
| 2.51 | $-CH_2CH_2CF=CF_2$ | 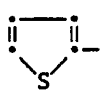 | S | O | |

Tabelle 2 (Fortsetzung)

| Verb. Nr. | $R_1$ | $R_2$ | $X_1$ | $X_2$ | Physikalische Daten |
|---|---|---|---|---|---|
| 2.52 | $-CH_2CH_2CF=CF_2$ | 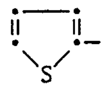 | S | S | |
| 2.53 | H | 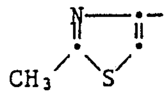 | S | S | Smp.>221°C Zers. |
| 2.54 | H | 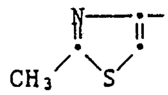 | S | O | Smp.>250°C |
| 2.55 | $-CHF_2$ | 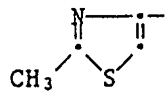 | S | S | Smp.156-158°C |
| 2.56 | $-CHF_2$ | 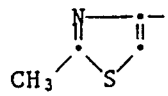 | S | O | Smp.191-193°C |
| 2.57 | $-CH_2CH_2CF=CF_2$ | 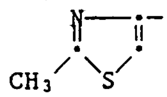 | S | S | Smp.136-138°C |
| 2.58 | $-CH_2CH_2CF=CF_2$ | 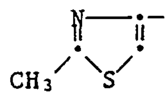 | S | O | Smp.150-153°C |

Tabelle 3, Oxa- und Thiazol-5-yl-Derivate

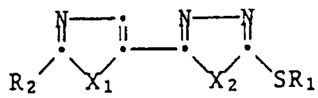

| Verb. Nr. | $R_1$ | $R_2$ | $X_1$ | $X_2$ | Physikalische Daten |
|---|---|---|---|---|---|
| 3.1 | H | H | S | O | Smp. 208-211°C |
| 3.2 | H | H | S | S | Smp. 186-188°C |
| 3.3 | H | $CH_3$ | S | O | |
| 3.4 | H | $CH_3$ | S | S | |
| 3.5 | H | $CH_3$ | O | O | |
| 3.6 | H | $CH_3$ | O | S | |
| 3.7 | $-CHF_2$ | $CH_3$ | S | O | |
| 3.8 | $-CHF_2$ | $CH_3$ | S | S | |
| 3.9 | $-CH_2CH_2CF=CF_2$ | $CH_3$ | S | O | |
| 3.10 | $-CH_2CH_2CF=CF_2$ | $CH_3$ | S | S | |
| 3.11 | H | $-NH_2$ | S | O | |
| 3.12 | H | $-NH_2$ | S | S | |
| 3.13 | $-CHF_2$ | $-NH_2$ | S | O | |
| 3.14 | -CHF2 | $-NH_2$ | S | S | |
| 3.15 | $-CH_2CH_2CF=CF_2$ | $CF_2=CFCH_2CH_2NH-$ | S | O | |
| 3.16 | $-CH_2CH_2CF=CF_2$ | $CF_2=CFCH_2CH_2NH-$ | S | S | |
| 3.17 | $-CHF_2$ | H | S | O | Oel |
| 3.18 | $-CHF_2$ | H | S | S | Smp. 107-109°C |
| 3.19 | $-CH_2CH_2CF=CF_2$ | H | S | O | Oel |
| 3.20 | $-CH_2CH_2CF=CF_2$ | H | S | S | Smp. 72-74°C |

2. Formulierungsbeispiele für flüssige Wirkstoffe der Formel I (% = Gewichtsprozent)

| 2.1 Emulsions-Konzentrate | a) | b) | c) |
|---|---|---|---|
| Wirkstoff aus den Tabellen 1-3 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2 Lösungen | a) | b) | c) | d) |
|---|---|---|---|---|
| Wirkstoff aus den Tabellen 1-3 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190° C) | - | - | 94 % | - |
| (MG = Molekulargewicht) | | | | |

Die Lösungen sind zur Anwendung in Form kleinster Tropfen geeignet.

| 2.3 Granulate | a) | b) |
|---|---|---|
| Wirkstoff aus den Tabellen 1-3 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) |
|---|---|---|
| Wirkstoff aus den Tabellen 1-3 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| 2.5 Spritzpulver | a) | b) | c) |
|---|---|---|---|
| Wirkstoff aus den Tabellen 1-3 | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6 Emulsions-Konzentrat | |
|---|---|
| Wirkstoff aus den Tabellen 1-3 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.7 Stäubemittel | a) | b) |
|---|---|---|
| Wirkstoff aus den Tabellen 1-3 | 5 % | 8 % |
| Talkum | 95 % | - |
| Kaolin | - | 92 % |

Man erhält anwendungsfertige Stäubemittel, indem der Wirkstoff mit den Trägern vermischt und auf einer geeigneten Mühle vermahlen wird.

| 2.8 Extruder Granulat | |
|---|---|
| Wirkstoff aus den Tabellen 1-3 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9 Umhüllungs-Granulat | |
|---|---|
| Wirkstoff aus den Tabellen 1-3 | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.10 Suspensions-Konzentrat | |
|---|---|
| Wirkstoff aus den Tabellen 1-3 | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

3.1 Wirkung gegen Meloidogyne incognita auf Tomaten

Eier von Meloidogyne incognita werden in Sand eingemischt. Dieses Gemisch wird dann in 200 ml fassende Tontöpfe eingebracht (5000 Eier pro Topf). Am gleichen Tag wird pro Topf eine 3 Wochen alte Tomatenpflanze gepflanzt und der formulierte Wirkstoff mittels Drenchapplikation in die Töpfe hineingegeben (0,0006 % Aktivsubstanz bezogen auf das Bodenvolumen). Die eingetopften Pflanzen werden nun bei einer Temperatur von 26±1 °C und einer relativen Luftfeuchtigkeit von 60 % in einem Gewächshaus aufgestellt. Nach Ablauf von 4 Wochen erfolgt eine Evaluierung durch Untersuchung der Pflanzen auf Wurzelgallbildung nach dem sogenannten Wurzelgallen-Index ("Root-Knot Index").

Verbindungen aus den Tabellen 1-3 zeigen gegen Meloidogyne incognita durch weitgehende Reduktion der Wurzelgallbildung gute Wirksamkeit. Unbehandelte jedoch infizierte Kontrollpflanzen weisen dagegen starke Wurzelgallbildung auf (= 100 %). So hemmen z.B. die Verbindungen Nr. 2.6, 2.8, 2.10, 2.12, 2.38 und 2.39 im obigen Versuch die Wurzelgallbildung fast vollständig (0-10 % Restbefall).

3.2. Pythium ultimum auf Beta vulgaris (Zuckerrübe, cv. Kleinwanzleben Monogerm) und auf Pythium ultimum auf Zea mays (Mais, cv. Sweet Corn)

Testprinzip: Bodenpilz: protektiv lokale Bodenapplikation.

Testmethode: Myzel von Pythium ultimum wird mit Erde gemischt (500 ml Myzelsuspension auf 10 Liter Erde) und das Pilz-Erdgemisch in 250 ml Plastikschalen abgefüllt. Nach einer 4-tägigen Inkubation bei 10 °C werden pro Schale 10 Körner der zu prüfenden Pflanze (Mais oder Zuckerrübe) gesteckt. Am nächsten Tag werden die so vorbereiteten Schalen mit je 50 ml, aus 25 % Spritzpulver und Wasser hergestellten Spritzlösungen mit 20; 6; 2; 0,6; 0,2; 0,06 und 0,02 ppm AS übergossen. Nach einer 7-tägigen Inkubationsphase bei 10 °C und einer anschliessenden 4-tägigen Inkubationsphase bei 22 °C wird die Wirkung der Testsubstanzen durch zahlenmässige Bestimmung des Auflaufs der Testpflanzen nach der folgenden Klassierung bewertet:

| mikrobizide Aktivität % | Bewertungsnote |
|---|---|
| > 95 | 1 |
| 80-95 | 3 |
| 50-80 | 6 |
| 0-50 | 9 (= wirkungslos) |

Verbindungen aus den Tabelle 1 - 3 wie z.B. 2.6, zeigen gegen Pythium ultimum gute Wirkung.

Beispiel 3.3: Wirkung gegen Rhizocotonia solani (Bodenpilz auf Reispflanzen)

Protektiv-lokale Bodenapplikation

12 Tage alte Reispflanzen werden mit einer aus einer Formulierung des Wirkstoffes hergestellten Spritzbrühe (0,002 % Aktivsubstanz), ohne oberirdische Pflanzenteile zu kontaminieren, angegossen. Zur Infizierung der behandelten Pflanzen wird eine Suspension von Myzel und Sklerotien von R. solani auf die Bodenoberfläche gegeben. Nach 6-tägiger Inkubation bei 27°C (Tag), bzw. 23°C (Nacht) und 100 % rel. Luftfeuchtigkeit (Feuchtkasten) in der Klimakammer wird der Pilzbefall auf Blattscheide, Blättern und Stengel beurteilt.

Nach Behandlung mit dem Wirkstoff trat kein Befall auf.

**Ansprüche**

1. 2-Mercapto-5-thiazol-(2-, 4- oder 5-yl)-1,3,4-oxa- und -thiadiazole und 2-Mercapto-5-oxazol-(2-, 4- oder 5-yl)-1,3,4-oxa- und -thiadiazole der Formel I

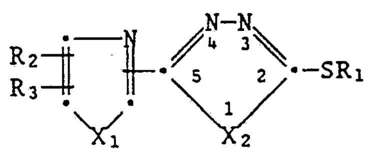

(I)

in welcher bedeuten:
$X_1$ und $X_2$ unabhängig voneinander Sauerstoff oder Schwefel $R_1$ unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl, $R_2$, $R_3$ unabhängig voneinander Wasserstoff, $C_1$-$C_5$-Alkyl, unsubstituiertes oder durch Halogen substituiertes $C_3$-$C_7$ Alkenyl, unsubstituiertes oder durch $C_1$-$C_3$-Alkyl, $C_1$-$C_3$-Alkoxy oder Halogen gleich oder verschieden substituiertes Phenyl, den Rest -$NR_4R_5$, wobei $R_4$ und $R_5$ unabhängig voneinander für Wasserstoff, $C_1$-$C_5$-Alkyl, unsubstituiertes oder durch Halogen substituiertes $C_3$-$C_7$-Alkenyl stehen, oder einer der beiden Substituenten $R_2$ oder $R_3$ auch einen der Heterocyclen Pyridyl, Thienyl, Furyl oder Thiazolyl bedeutet, der unsubstituiert oder durch $C_1$-$C_3$-Alkyl, $C_1$-$C_3$-Alkoxy oder Halogen ein oder mehrfach gleich oder verschieden substituiert sein kann, sowie Salze dieser Verbindungen.

2. Verbindungen der Formel I gemäss Anspruch 1, worin $X_1$ und $X_2$ unabhängig voneinander O oder S, und $R_1$ durch Halogen substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl bedeuten; $R_2$ Wasserstoff $C_1$-$C_3$ Alkyl ist; $R_3$ Wasserstoff, $C_1$-$C_3$-Alkyl, unsubstituiertes oder durch $C_1$-$C_3$-Alkyl, $C_1$-$C_3$-Alkoxy oder Halogen gleich oder verschieden substituiertes Phenyl oder den Rest -$NR_4R_5$ bedeutet, oder einer der beiden Substituenten $R_2$ oder $R_3$ auch einen der Hetero cyclen Pyridyl, Thienyl, Furyl oder Thiazolyl bedeutet, der unsubstituiert oder wahlweise durch $C_1$-$C_3$-Alkyl, $C_1$-$C_3$-Alkoxy oder Halogen gleich oder verschieden ein- oder zweifach substituiert sein kann.

3. Verbindungen der Formel I gemäss Anspruch 2 in welchen bedeuten: $X_1$ und $X_2$ unabhängig voneinander Sauerstoff oder Schwefel $R_1$ unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl, $R_2$ und $R_3$ unabhängig voneinander Wasserstoff, $C_1$-$C_5$-Alkyl, unsubstituiertes oder durch Halogen substituiertes $C_3$-$C_7$-Alkenyl, unsubstituiertes oder durch $C_1$-$C_3$-Alkyl, $C_1$-$C_3$-Alkoxy oder Halogen gleich oder verschieden substituiertes Phenyl, oder einer der beiden Substituenten $R_2$ oder $R_3$ auch einen der Heterocyclen Pyridyl, Thienyl, Furyl oder Thiazolyl bedeutet, der unsubstituiert oder durch $C_1$-$C_3$-Alkyl, $C_1$-$C_3$-Alkoxy oder Halogen ein oder mehrfach gleich oder verschieden substituiert sein kann.

4. Verbindungen gemäss Anspruch 3 bei welchen $X_1$ und $X_2$ unabhängig voneinander Sauerstoff oder

Schwefel $R_1$ unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl, $R_2$ und $R_3$ unabhängig voneinander Wasserstoff, $C_1$-$C_3$-Alkyl, unsubstituiertes oder durch $C_1$-$C_3$-Alkyl, $C_1$-$C_3$-Alkoxy oder Halogen substituiertes Phenyl, ferner Pyridyl, Thienyl, Furyl oder Thiazolyl bedeuten, sowie Salze dieser Verbindungen.

5. 2-Mercapto-5-thiazol-(2-, 4- oder 5-yl)-1,3,4-oxadiazole der Formel Ia

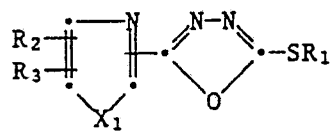

(Ia)

gemäss Anspruch 4, worin $R_1$ Difluormethyl, 3,4,4-Trifluor-3-buten, Cyanomethyl, 1,1,2,2-Tetrafluroäthyl oder Propargyl, $R_2$ und $R_3$ unabhängig voneinander Wasserstoff oder Methyl bedeuten.

6. Ein 2-Mercapto-5-thiazol-(2-, 4- oder 5-yl)-1,3,4-oxadiazol gemäss Anspruch 1, ausgewählt aus der Gruppe, bestehend aus
2-Difluormethylthio-5-(thiazol-2-yl)-1,3,4-oxadiazol,
2-(3,4,4-Trifluor-3-butenylthio)-5-(thiazol-2-yl)-1,3,4-oxadiazol,
2-Difluormethylthio-5-(2-methyl-thiazol-4-yl)-1,3,4-oxadiazol,
2-(3,4,4-Trifluor-3-butenylthio)-5-(2-methyl-thiazol-4-yl)-1,3,4-oxadiazol,
2-Difluormethylthio-5-(thiazol-5-yl)-1,3,4-oxazol und
2-(3,4,4-Trifluor-3-butenylthio)-5-(thiazol-5-yl)-1,3,4-oxadiazol.

7. Das 2-(3,4,4-Trifluor-3-butenylthio)-5-[2-(pyrid-3-yl)-thiazol-4-yl]-1,3,4-oxadiazol gemäss Anspruch 1.

8. 2-Mercapto-5-oxazol-(2-, 4- oder 5-yl)-1,3,4-thiadiazole der Formel Ib

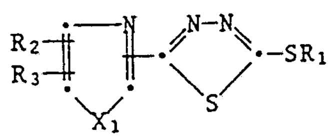

(Ib)

gemäss Anspruch 4, worin $R_1$ Difluormethyl, 3,4,4-Trifluor-3-buten, 1,1,2,2-Tetrafluoräthyl, Cyanomethyl, Allyl, Propargyl oder 2-Bromalkyl, $R_2$ und $R_3$ unabhängig voneinander Wasserstoff oder Methyl bedeuten.

9. Ein 2-Mercapto-5-oxazol-(2-, 4- oder 5-yl)-1,3,4-thiadiazol gemäss Anspruch 1, ausgewählt aus einer Gruppe, bestehend aus
2-Difluormethylthio-5-(thiazol-2-yl)-1,3,4-thiadiazol,
2-(3,4,4-Trifluor-3-butenylthio)-5-(thiazol-2-yl)-1,3,4-thiadiazol,
2-Difluormethylthio-5-(2-methyl-thiazol-4-yl)-1,3,4-thiadiazol,
2-(3,4,4-Trifluor-3-butenylthio)-5-(2-methyl-thiazol-4-yl)-1,3,4-thiadiazol,
2-Difluormethylthio-5-(thiazol-5-yl)-1,3,4-thiadiazol und
2-(3,4,4-Trifluor-3-butenylthio)-5-(thiazol-5-yl)-1,3,4-thiadiazol.

10. Verfahren zur Herstellung der Verbindungen der Formel I gemäss Anspruch 1, dadurch gekennzeichnet, dass man

a) in einer Kondensationsreaktion eine Verbindung der Formel II

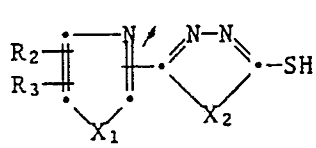

(II)

oder eine Verbindung der Formel III

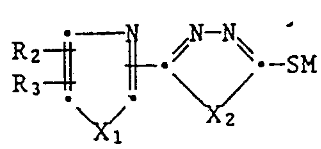

(III)

mit einer Verbindung der Formel IV

Hal-$R_1$ (IV)

in einem inerten Lösungsmittel oder Lösungsmittel-Gemisch bei Raumtemperatur oder bei erhöhter Temperatur gegebenenfalls in Gegenwart eines Katalysators und gegebenenfalls bei erhöhtem Druck umsetzt, wobei die Umsetzung einer Verbindung der Formel II in Gegenwart einer Base verläuft, oder

b) in einer Anlagerungsreaktion eine Verbindung der Formel II

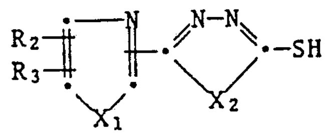

(II)

mit einer Verbindung der Formel V

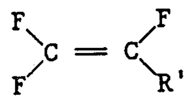

(V)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ic

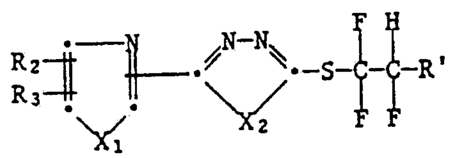

(Ic)

oder zu einer Verbindung der Formel Id

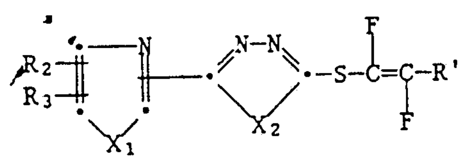

(Id)

führt, wobei in den vorstehend genannten Formeln II, III, Ic, Id, IV und V M für ein Alkalimetall oder für Ammonium steht, Hal Halogen, vorzugsweise Chlor, Brom oder Jod bedeutet, und R′ Fluor oder Trifluormethyl darstellt, während $R_1$, $R_2$, $R_3$, $X_1$ und $X_2$ die unter Formel I angegebenen Bedeutungen besitzen.

11. Schädlingsbekämpfungsmittel zur Bekämpfung oder Verhütung des Befalls von Pflanzen durch Nematoden und Pilze, dadurch gekennzeichnet, dass es als aktive Komponente mindestens eine Verbindung der Formel I, gemäss Anspruch 1 enthält.

12. Mittel gemäss Anspruch 11, dadurch gekennzeichnet, dass es als aktive Komponente mindestens eine Verbindung der Formel I gemäss der Ansprüche 2 bis 8 enthält.

13. Mittel gemäss Anspruch 11, dadurch gekennzeichnet, dass es als aktive Komponente eine Verbindung ausgewählt aus einer Gruppe, bestehend aus 2-Mercapto-5-thiazol-(2-, 4- oder 5-yl)-1,3,4-oxadiazol enthält.

14. Mittel gemäss Anspruch 11, dadurch gekennzeichnet, dass es als aktive Komponente eine Verbindung ausgewählt aus einer Gruppe, bestehend aus
2-Difluormethylthio-5-(thiazol-2-yl)-1,3,4-thiadiazol,
2-(3,4,4-Trifluor-3-butenylthio)-5-(thiazol-2-yl)-1,3,4-thiadiazol,
2-Difluormethylthio-5-(2-methyl-thiazol-4-yl)-1,3,4-thiadiazol,
2-(3,4,4-Trifluor-3-butenylthio)-5-(2-methyl-thiazol-4-yl)-1,3,4-thiadiazol,
2-Difluormethylthio-5-(thiazol-5-yl)-1,3,4-thiadiazol und
2-(3,4,4-Trifluor-3-butenylthio)-5-(thiazol-5-yl)-1,3,4-thiadiazol,
enthält.

15. Verfahren zur Herstellung eines agrochemischen Mittels, dadurch gekennzeichnet, dass mindestens eine Verbindung der Formel I gemäss Anspruch 1 mit geeigneten festen oder flüssigen Zusatzstoffen und Tensiden innig vermischt.

16. Verfahren zur Bekämpfung oder Verhütung eines Befalls von Kulturpflanzen durch Nematoden und Pilze, dadurch gekennzeichnet, dass man eine Verbindung der Formel I gemäss einem der Ansprüche 1 bis 9 auf die Pflanze oder deren Standort appliziert.

17. Verwendung von Verbindungen der Formel I gemäss Anspruch 1 zur Bekämpfung und/oder zur präventiven Verhütung eines Befalls von Pflanzen durch Nematoden und Pilze.

Patentansprüche für folgenden Vertragsstaat: ES

1. Verfahren zur Herstellung von 2-Mercapto-5-thiazol-(2-, 4- oder 5-yl)-1,3,4-oxa- und -thiadiazolen und 2-Mercapto-5-oxazol-(2-, 4- oder 5-yl)-1,3,4-oxa- und -thiadiazolen der Formel I

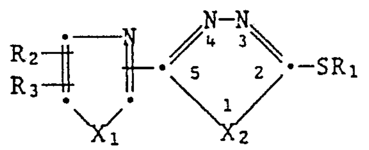

(I)

in welcher bedeuten:

$X_1$ und $X_2$ unabhängig voneinander Sauerstoff oder Schwefel $R_1$ unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl, $R_2$, $R_3$ unabhängig voneinander Wasserstoff, $C_1$-$C_5$-Alkyl, unsubstituiertes oder durch Halogen substituiertes $C_3$-$C_7$ Alkenyl, unsubstituiertes oder durch $C_3$-$C_3$-Alkyl, $C_1$-$C_3$-Alkoxy oder Halogen gleich oder verschieden substituiertes Phenyl, den Rest $-NR_4R_5$, wobei $R_4$ und $R_5$ unabhängig voneinander für Wasserstoff, $C_1$-$C_5$-Alkyl, unsubstituiertes oder durch Halogen substituiertes $C_3$-$C_7$-Alkenyl stehen, oder einer der beiden Substituenten $R_2$ oder $R_3$ auch einen der Heterocyclen Pyridyl, Thienyl, Furyl oder Thiazolyl bedeutet, der unsubstituiert oder durch $C_1$-$C_3$-Alkyl, $C_1$-$C_3$-Alkoxy oder Halogen ein oder mehrfach gleich oder verschieden substituiert sein kann, dadurch gekennzeichnet, dass man

a) in einer Kondensationsreaktion eine Verbindung der Formel II

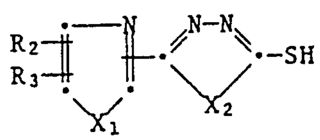

(II)

oder eine Verbindung der Formel III

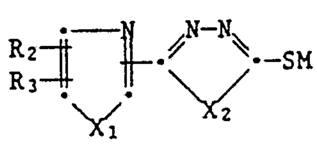

(III)

mit einer Verbindung der Formel IV

$Hal-R_1$ (IV)

in einem inerten Lösungsmittel oder Lösungsmittel-Gemisch bei Raumtemperatur oder bei erhöhter Temperatur gegebenenfalls in Gegenwart eines Katalysators und gegebenenfalls bei erhöhtem Druck umsetzt, wobei die Umsetzung einer Verbindung der Formel II in Gegenwart einer Base verläuft, oder

b) in einer Anlagerungsreaktion eine Verbindung der Formel II

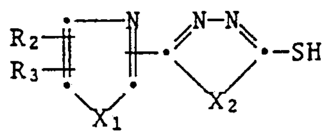

(II)

mit einer Verbindung der Formel V

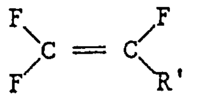

(V)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ic

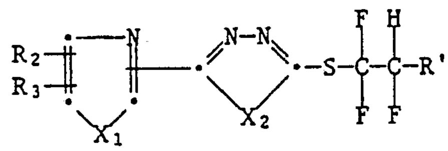

(Ic)

oder zu einer Verbindung der Formel Id

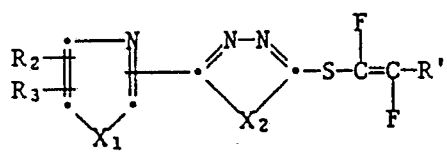

(Id)

führt, wobei in den vorstehend genannten Formeln II, III, Ic, Id, IV und V M für ein Alkalimetall oder für Ammonium steht, Hal Halogen, vorzugsweise Chlor, Brom oder Jod bedeutet, und R′ Fluor oder Trifluormethyl darstellt, während $R_1$, $R_2$, $R_3$, $X_1$ und $X_2$ die unter Formel I angegebenen Bedeutungen besitzen.

2. Verfahren gemäss Anspruch 1, dadurch gekennzeichnet, dass $X_1$ und $X_2$ unabhängig voneinander O oder S, und $R_1$ durch Halogen substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl bedeuten; $R_2$ Wasserstoff $C_1$-$C_3$ Alkyl ist; $R_3$ Wasserstoff, $C_1$-$C_3$-Alkyl, unsubstituiertes oder durch $C_1$-$C_3$-Alkyl, $C_1$-$C_3$-Alkoxy oder Halogen gleich oder verschieden substituiertes Phenyl oder den Rest $-NR_4R_5$ bedeutet, oder einer der beiden Substituenten $R_2$ oder $R_3$ auch einen der Heterocyclen Pyridyl, Thienyl, Furyl oder Thiazolyl bedeutet, der unsubstituiert oder wahlweise durch $C_1$-$C_3$-Alkyl, $C_1$-$C_3$-Alkoxy oder Halogen gleich oder verschieden ein- oder zweifach substituiert sein kann.

3. Verfahren gemäss Anspruch 2, dadurch gekennzeichnet, dass $X_1$ und $X_2$ unabhängig voneinander Sauerstoff oder Schwefel $R_1$ unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl, $R_2$ und $R_3$ unabhängig voneinander Wasserstoff, $C_1$-$C_5$-Alkyl, unsubstituiertes oder durch Halogen substituiertes $C_3$-$C_7$-Alkenyl, unsubstituiertes oder durch $C_1$-$C_3$-Alkyl, $C_1$-$C_3$-Alkoxy oder Halogen gleich oder verschieden substituiertes Phenyl, oder einer der beiden Substituenten $R_2$ oder $R_3$ auch einen der Heterocyclen Pyridyl, Thienyl, Furyl oder Thiazolyl bedeuten, der unsubstituiert oder durch $C_1$-$C_3$-Alkyl, $C_1$-$C_3$-Alkoxy oder Halogen ein oder mehrfach gleich oder verschieden substituiert sein kann.

4. Verfahren gemäss Anspruch 3, dadurch gekennzeichnet, dass $X_1$ und $X_2$ unabhängig voneinander Sauerstoff oder Schwefel $R_1$ unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl, $R_2$ und $R_3$ unabhängig voneinander Wasserstoff, $C_1$-$C_3$-Alkyl, unsubstituiertes oder durch $C_1$-$C_3$-Alkyl, $C_1$-$C_3$-Alkoxy oder Halogen substituiertes Phenyl, ferner Pyridyl, Thienyl, Furyl oder Thiazolyl bedeuten.

5. Verfahren zur Herstellung von 2-Mercapto-5-thiazol-(2-, 4- oder 5-yl)-1,3,4-oxadiazolen der Formel Ia

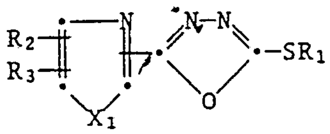

(Ia)

gemäss Anspruch 4, dadurch gekennzeichnet, dass $R_1$˙Difluormethyl, 3,4,4-Trifluor-3-buten, Cyanomethyl, 1,1,2,2-Tetrafluroäthyl oder Propargyl, $R_2$ und $R_3$ unabhängig voneinander Wasserstoff oder Methyl bedeuten.

6. Verfahren zur Herstellung von
2-Difluormethylthio-5-(thiazol-2-yl)-1,3,4-oxadiazol,
2-(3,4,4-Trifluor-3-butenylthio)-5-(thiazol-2-yl)-1,3,4-oxadiazol,
2-Difluormethylthio-5-(2-methyl-thiazol-4-yl)-1,3,4-oxadiazol,
2-(3,4,4-Trifluor-3-butenylthio)-5-(2-methyl-thiazol-4-yl)-1,3,4-oxadiazol,
2-Difluormethylthio-5-(thiazol-5-yl)-1,3,4-oxazol und
2-(3,4,4-Trifluor-3-butenylthio)-5-(thiazol-5-yl)-1,3,4-oxadiazol gemäss Anspruch 1.

7. Verfahren zur Herstellung von 2-(3,4,4-Trifluor-3-butenylthio)-5-[2-(pyrid-3-yl)-thiazol-4-yl]-1,3,4-oxadiazol gemäss Anspruch 1.

8. Verfahren zur Herstellung von 2-Mercapto-5-oxazol-(2-, 4- oder 5-yl)-1,3,4-thiadiazolen der Formel Ib

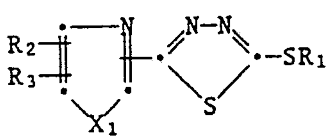

(Ib)

gemäss Anspruch 4, dadurch gekennzeichnet, dass $R_1$ Difluormethyl, 3,4,4-Trifluor-3-buten, 1,1,2,2-Tetrafluoräthyl, Cyanomethyl, Allyl, Propargyl oder 2-Bromalkyl, $R_2$ und $R_3$ unabhängig voneinander Wasserstoff oder Methyl bedeuten.

9. Verfahren zur Herstellung von
2-Difluormethylthio-5-(thiazol-2-yl)-1,3,4-thiadiazol,
2-(3,4,4-Trifluor-3-butenylthio)-5-(thiazol-2-yl)-1,3,4-thiadiazol,
2-Difluormethylthio-5-(2-methyl-thiazol-4-yl)-1,3,4-thiadiazol,
2-(3,4,4-Trifluor-3-butenylthio)-5-(2-methyl-thiazol-4-yl)-1,3,4-thiadiazol,
2-Difluormethylthio-5-(thiazol-5-yl)-1,3,4-thiadiazol und
2-(3,4,4-Trifluor-3-butenylthio)-5-(thiazol-5-yl)-1,3,4-thiadiazol gemäss Anspruch 1.

Europäisches Patentamt

# EUROPÄISCHER RECHERCHENBERICHT

Nummer der Anmeldung

EP 90810194.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
|---|---|---|---|
| X | DD - A1 - 258 814 (VEB BITTERFELD) * Beispiel 9 * -- | 1 | C 07 D 417/04<br>C 07 D 413/04<br>C 07 D 417/14<br>C 07 D 413/14<br>A 01 N 43/82 |
| D,A | DE - A1 - 2 361 613 (PEPRO) * Ansprüche 1,2 * -- | 1,11-17 | |
| D,A | US - A - 3 770 754 (PARSONS) * Anspruch 1; Spalte 3, Zeilen 10-29 * -- | 1,11-17 | |
| D,A | US - A - 4 454 147 (DI MENNA) * Zusammenfassung * ---- | 1,11-17 | |

RECHERCHIERTE SACHGEBIETE (Int. Cl.⁵)

C 07 D 417/00
C 07 D 413/00

Der vorliegende Recherchenbericht wurde für alle Patentansprüche erstellt.

| Recherchenort | Abschlußdatum der Recherche | Prüfer |
|---|---|---|
| WIEN | 04-05-1990 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82